# EUROPEAN PATENT SPECIFICATION

(11) **EP 0 308 670 B1**
(45) Date of publication and mention of the grant of the patent: **04.05.1994**
(21) Application number: 88113754.1
(22) Date of filing: 24.08.1988
(51) Int. Cl.: G01N 33/84, G01N 31/22, G01N 33/52

(54) **Simultaneous assay for calcium and phosphorus**
Simultanbestimmung von Calcium und Phosphor
Essai simultané pour le calcium et le phosphore

(30) Priority: 22.09.1987 US 99587
(43) Date of publication of application: 29.03.1989
(73) Proprietor: ABBOTT LABORATORIES, Abbott Park, Illinois 60064-3500 (US)
(72) Inventor: Bates, Diane Marian, Roanoke, TX 76262 (US); Czervionke, Robert Leo, Grapevine, TX 76051 (US)
(74) Representative: Modiano, Guido, Dr.-Ing.

(56) References cited:
- CHEMICAL ABSTRACTS, vol. 96, no. 1, 04 January 1982, Columbus, OH (US); O.I. VERSHININA et al., p. 290, no. 3208s#
- CHEMICAL ABSTRACTS, vol. 79, no. 9, 03 September 1973, Columbus, OH (US); Y. MIYAHARA et al., p. 133, no. 50486q#
- CHEMICAL ABSTRACTS, vol. 99, no. 1, 04 July 1983, Columbus, OH (US); J.F. VAN STADEN, p. 397, no. 4165z#
- CHEMICAL ABSTRACTS, vol. 89, no. 13, 25 September 1978, Columbus, OH (US); L. HAMBLETON, p. 632, no. 105992r#
- CHEMICAL ABSTRACTS, vol. 87, no. 17, 24 October 1977, Columbus, OH (US); L.G. HAMBLETON, p. 519, no. 132412s#
- CHEMICAL ABSTRACTS, vol. 81, no. 25, 23 December 1974, Columbus, OH (US); W.G. BRYDON, p. 238, no. 165738g#
- 9-BIOCHEM. METHODS, vol. 82, 1975; no. 1352242#
- ADV. AUTOM. ANAL., Technician Int. Congr. 1972 (pub.1973), vol. 1, Mediad Inc., Tarrytown, NY (US); pp. 173-178#

## Description

### BACKGROUND OF THE INVENTION

The present invention relates to the simultaneous measurement of a plurality of substrates with a single reagent by monitoring concurrent reactions which produce changes in the electromagnetic radiation absorbance characteristics of the sample. In one aspect, the invention relates to the simultaneous measurement of calcium and phosphorus (inorganic phosphate) in blood serum by monitoring two concurrent reactions at two or more different wavelengths.

In the field of diagnostics, various assays are designed to identify or quantify a substrate which may be present in a sample material. Unfortunately the assay is usually only specific to one type of substrate even though it may be desirable to diagnose more than one substrate for any given sample. This leads to multiple testing on the same sample which increases diagnosis cost and decreases efficiency. It is therefore desirable to develop diagnostic testing which can identify or quantify multiple substrates in an efficient manner.

For example, calcium and phosphorus are two tests performed routinely in the clinical chemistry laboratory. Analysis of calcium by direct spectrophotometric methods generally rely on the formation of a colored complex between calcium and an organic molecule. The o-cresolphthalein complexone has been most commonly employed in recent years. It quantitates a red complex at 570 to 575 nm at pH 10 to 12, and eliminates magnesium interference with 8-hydroxyquinoline compounds. Alizarin, arsenazo-III, chlorophosphonazo-III, and methylthymol blue are used in other assays to form colored complexes. Atomic absorption spectroscopy, the calcium reference method, is employed in some labs. (See, Kaplan, L.A., and Pesce, A.J., Clinical Chemistry, 1984, p. 1052).

Analysis of phosphorus is typically based on the reaction of phosphate ions with molybdate to form phosphomolybdate complexes. The complexes are either measured directly at 340 nm and acid pH, or after reduction to molybdenum blue at 660 nm. (Kaplan, p. 1072.)

The calcium and phosphorus assays mentioned above are performed with separate reagents in separate cuvettes. This costs the clinical chemistry lab time and money. By combining the two tests into one test the lab would be able to realize an increase in productivity and also a cost savings.

Combining the two tests is not a straightforward task. Conditions must be selected that allow precise measurement of both substrates. For example, the combination of the calcium o-cresolphthalein complexone (pH 10-12) and the unreduced molybdate phosphorus procedure (pH about 1) cannot be used due to the extremes of pH. In addition, the quinoline compounds used to eliminate magnesium interference in the calcium assay negate the combination because they absorb near the phosphomolybdate complex and thus may interfere with the phosphorus assay.

One way of combining two assays in a single reaction vessel is to do a sequential assay. In a sequential assay, reagent for the first assay is added to the vessel and the reaction proceeds. At some later time a concentration is determined for the first component. Then a second reagent, which either quenches the first reaction or is added after the first reaction is complete, is added to the vessel to trigger a reaction with the second component. At some later time the concentration of the second component is determined. These reactions can either be monitored at the same wavelength or at different wavelengths (either through the use of filter wheels or diode arrays). U.S. Patent 4,425,427 to Luderer discloses sequential assays for glucose and urea as well as a few simultaneous assays for analytes other than the calcium and phosphorus pair.

An automated procedure for simultaneously determining serum calcium (cresolphthalein complex) and inorganic phosphorus (molybdate complex) in an AutoAnalyzer is known from Chem. Abstr., vol. 79, 1973, no. 50486q. A 3-channel flow-injection procedure for the simultaneous determination of protein N, phosphorus (Mo blue complex) and calcium (cresolphthalein complex) was disclosed by Chem. Abstr., vol. 99, 1983, no. 4165z.

A semiautomated method for the simultaneous determination of protein N, phosphorus (molybdovanadophosphate complex) and calcium (cresolphthalein complex) in an AutoAnalyzer II system is known from Chem. Abstr., vol. 89, 1978, no. 105992r. A semiautomated method for the simultaneous determination of protein N, phosphorus (molybdovanadophosphate complex) and calcium (cresolphthalein complex) is also known from Chem. Abstr., vol. 87, 1977, no. 132412s. A continuous flow technique for the simultaneous determination of calcium and inorganic phosphate (phosphomolybdate-methyl green complex) in serum and urine is known from Chem. Abstr., vol. 81, 1974, no. 165738g.

U.S. Patent 3,925,162 describes the simultaneous measurement of enzyme activity in body fluids. In this approach the substrate for each of the enzymes to be identified are added to a reaction medium with other reagents and changes in the absorbance or fluorescence of the resulting reaction system are measured. The present invention utilizes an approach where a single reagent system is used to simultaneously identify or quantify substrate by monitoring the electromagnetic signal of the reaction mixture.

### SUMMARY OF THE INVENTION

The present invention is directed toward a method for the simultaneous determination of calcium and phosphorus (inorganic phosphate) substrates with a single reagent system in a reaction mixture. The method comprises adding a reagent system containing a reactant for each of the substrates to be determined, each reactant being selected such that it is capable of giving a unique electromagnetic radiation absorbance for the particular substrate and permits calculation of both substrate concentrations. The substrates are reacted with their respective reagent under conditions such that the chemistries take place simultaneously. The concentration of the substrates is determined by measuring changes in absorbance or fluorescence of the resulting reaction mixture at a plurality of wavelengths which are characteristic for each of the substrates to be determined.

In another aspect the present invention is a method for the simultaneous determination of calcium and phosphorus substrates with a single reagent system in a reaction mixture by adding a reagent system containing a chromophore for each of the substrates to be determined, each chromophore being selected such that it is capable of giving a unique absorbance band for the particular substrate and allows the determination of the other substrate. The substrates are reacted with their respective chromophore under conditions such that the reaction takes place simultaneously. The concentration of the substrates is determined by measuring changes in absorbance or fluorescence of the resulting reaction mixture at a plurality of wavelengths which are characteristic for each of the substrates to be determined.

Generally the reagent system for performing the simultaneous assay of calcium and phosphorus comprises an azo dye in a concentration sufficient to allow an endpoint determination of calcium, and an acidic, aqueous solution comprising a molybdate salt and detergent in a concentration sufficient to dissolve serum components and allow an endpoint determination of phosphorus.

A reagent system useful in performing the present simultaneous assay comprises chlorophosphonazo-III in a concentration sufficient to allow an endpoint determination of calcium; and sodium or ammonium molybdate, an acid (preferably sulfuric acid) in an amount sufficient to adjust the reagent system to about pH 2, and detergent in a concentration sufficient to dissolve serum components and allow an endpoint determination of phosphorus.

Another reagent system useful in performing the present simultaneous assay comprises chlorophosphonazo-III or arsenazo-III in a concentration sufficient to allow an endpoint determination of calcium; and ammonium molybdate, acetic acid/NaOH in an amount suffic to adjust the reagent system to about pH 4 and detergent in a concentration sufficient to dissolve serum components and allow an endpoint determination of phosphorus.

### DETAILED DESCRIPTION OF THE INVENTION

The present invention relates to a method for simultaneously measuring a plurality of substrates in a biological fluid. The subject method involves a single reagent addition to a sample for measurement of each of the substrates by monitoring several electromagnetic signals simultaneously.

The electromagnetic signals can be monitored simultaneously by a spectrophotometer, or spectrofluorometer. The measurement of changes in the reaction mixture can be carried out on any of the instruments by conventional procedures. The particular change in the system ,i.e., wavelength, is not critical, but it is preferable that the changes or differences in wavelength be as great as possible provided they can be monitored simultaneously.

In a simultaneous assay a reagent containing all the components for reaction with the substrates to be measured are added to the sample and the reactions are monitored by the instrument. Typically, a simultaneous assay is done in a single cuvette with a single reagent, eliminating the need for a second reagent dispense or other optional steps generally associated with multiple substrate assays.

A key to the design of a simultaneous assay is the selection of reagents that will allow the reactions to proceed simultaneously and permit accurate determination of both analytes in the clinically relevant range. Reactants are chosen for each of the substrates to be determined such that each is capable of giving a unique electromagnetic radiation absorbance for the particular substrate. A reactant can be a chromophore or indicator dye where the reaction will be monitored by spectral wavelength. For example, by choosing appropriate chromophores an assay can be developed that will measure calcium and phosphorus simultaneously as described below.

After the proper reactant is chosen the sample is added to the reagent system which contains the appropriate reactants. The reagent and sample are mixed such that each of the substrates is contacted with their respective reactant under conditions such that the reaction takes place simultaneously. The addition and mixing of the sample and reagent is monitored by instrumentation appropriate for the reaction taking place such as measuring changes in absorbance or fluorescence of the resulting reaction mixture at a plurality of wavelengths which are characteristic for each of the substrates to be determined.

Preferably the monitoring of the reaction mixture is begun as soon as the reagent and sample are intermixed. This allows for monitoring of changes in either the reaction rate or endpoint reaction change for the particular electromagnetic signal being monitored.

The subject methods allow for the simultaneous measurement of calcium and phosphorus (inorganic phosphate) in blood serum using a single reagent. The calcium and phosphorus reactions proceed at the same time, with measurement of the two different reactions monitored at two separate wavelengths by a spectrophotometer. The spectrophotometer employs a diode array detector having the capability of simultaneously monitoring many wavelengths.

Generally the reagent system for performing the simultaneous assay of calcium and phosphorus comprises an azo dye in a concentration sufficient to allow an endpoint determination of calcium, and an acidic, aqueous solution comprising a molybdate salt and detergent in a concentration sufficient to dissolve serum components and allow an endpoint determination of phosphorus.

In one aspect, the measurement of calcium is through the use of an azo dye such as chlorophosphonazo-III. The absorbance maximum is near 660 nm, with relatively little absorbance change at from about 330 to about 400 nm. This allows coupling of the calcium reagent with a pH 2 phosphorus reagent using a molybdate salt, and detergent which can be monitored at from about 330 to about 400 nm with no absorbance change at about 660 nm. In addition, the azo dye calcium indicator is ideal for combination with the pH 2 phosphorus reagent because, at pH 2 to 5, the dye exhibits sufficient molar absorptivity and essentially no interference due to magnesium (CRC Handbook of Organic Analytical REagents, K.L. Cheng, K. Ueno, T. Imamura, CRC Press, Boco Raton, FL, p. 173, Fig. 5, 1982). This allows magnesium interference in the calcium assay to be removed by pH choice and not by the addition of a compound which could interfere with the simultaneous phosphorus assay. The pH of the reagent system can be adjusted by the addition of an acid such as sulfuric acid.

The molybdate salt in the phosphorus assay is perferably sodium or ammonium molybdate. The phosphorus portion of this simultaneous assay reagent system method is similar to the procedure of Munoz, et al., Clin. Chem. 29:372, (1983), herein incorporated by reference, modified for use with an azo dye. Specifically, the modification involved substituting sulfuric acid for nitric acid because the latter may degrade an azo dye (CRC Handbook, p. 171).

In another approach for performing the simultaneous assay the calcium portion of the reagent system consists of an azo dye preferably chlorophosphonazo-III or arsenazo-III at about pH 4. The reaction is monitored at about 660 nm. There is little absorbance change at 340 or 364 nm so this reaction will not interfere with the phosphorus part of the assay. As above, the pH was chosen to negate magnesium interference.

The phosphorus or inorganic phosphate portion of this reagent system comprises an ammonium or sodium molybdate, detergent, and acetic acid, adjusted to a pH 4 with NaOH. Typical detergents which can be employed are Brij-35, a trademark of ICI Americas, Inc., Wilmington, Delaware, for polyoxyethylene (23) lauryl ether, and sodium dodecyl sulfate. The phosphorus method is similar to the procedure of Atkinson, et al., Biochimical et Biophysica Acta 320:195 (1973), herein incorporated by reference, modified by increasing the detergent concentration to dissolve serum components. Preferably, the sodium dodecyl sulfate is increased to solubilize the serum components. The reaction of phosphate with molybdate is monitored from about 330 to about 400, more preferably from about 340 to about 364 nm. There is insignificant absorbance change at 660 nm so this reaction will not interfere with the calcium part of the assay.

To further describe the instant invention the following examples are provided.

### EXAMPLE 1

### Calcium/Phosphorus Simultaneous Assay

The following procedure describes a method for performing a simultaneous assay for calcium and phosphorus by monitoring the endpoints of both the calcium and the phosphorus reactions. A single reagent system was made by preparing an aqueous solution of 20 mM sodium molybdate and 250 milliliters per liter (mL/L) 40% triethanolamine lauryl sulfate. The aqueous solution was adjusted to a pH of 2 with 17.8M H₂SO₄, diluted to volume with deionized water and 0.1 millimoles per liter (mM) of chlorophosphonazo-III was added.

Serum sample (human) was added to the reagent at a ratio of 1:25 and mixed. After two minutes the absorbance was read at 340 nm for phosphorus and at 660 nm for calcium. Another assay was performed exactly as described but the absorbance was read at 364 nm for phosphorus and 660 nm for calcium. Concentrations were calculated by comparison with standard curves.

### EXAMPLE 2

### Calcium/Phosphorus simultaneous Assay

The following procedure describes a method for performing a simultaneous assay for calcium and phosphorus by monitoring the endpoints of both the calcium and the phosphorus reactions. A single reagent system was made by preparing an aqueous solution of 2.9 mM ammonium heptamolybdate and 250 mL/L 40% triethanolamine lauryl sulfate. The aqueous solution was adjusted to a pH of 2 with 17.8M H₂SO4, diluted to volume with deionized water and 0.1 mM of chlorophosphonazo-III was added.

Serum sample (human) was added to the reagent at a ratio of 1:25 and mixed. After two minutes the absorbance was read at 340 nm for phosphorus and at 660 nm for calcium. Another assay was performed exactly as described but the absorbance was read at 364 nm for phosphorus and 660 nm for calcium. Concentrations were calculated by comparison with standard curves.

### EXAMPLE 3

### Calcium/Phosphorus simultaneous Assay

The following procedure describes a method for performing a simultaneous assay for calcium and phosphorus by monitoring the calcium and the phosphorus reaction endpoints. A reagent system was made by preparing an aqueous solution containing:
0.1 mN chlorophosphonazo-III
100 mL/L 4% Brij-35®
100 mL/L 10% sodium dodecyl sulfate
250 mL/L (1.3 %) ammonium heptamolybdate
550 mL/L 0.17M acetic acid, adjusted to pH 4 with NAOH
Serum sample (human) was added to the reagent at a ratio of 1:25 and mixed. The calcium reaction was read at 660 nm and phosphorus was determined at 340 nm, after 2 minutes. Another assay was performed exactly as described but the absorbance was read at 364 nm for phosphorus and 660 nm for calcium. Concentrations are calculated by comparison with standard curves.

## Claims (Claims for the following Contracting State(s): AT, BE, CH, DE, FR, GB, IT, LI, NL)

1. A method for the simultaneous determination of the concentration of calcium and phosphorus substrates in a single test sample with a single reagent system, said method comprising the steps of:
adding to said test sample in a single step said single reagent system containing a reactant for each of said calcium and phosphorus substrates, each reactant being selected such that it is capable of contributing to a discrete electromagnetic radiation signal for the respective calcium or phosphorus substrate, wherein the determination of calcium does not interfere with the determination of phosphorus, and the determination of phosphorus does not interfere with the determination of calcium;
reacting said test sample with said reagent system whereby said calcium and phosphorus substrates react simultaneously with their respective reactants;
monitoring changes in absorbance or fluorescence of the resulting reaction mixture at two or more wavelengths at least one of which which is characteristic of said calcium substrate and at least one of which is characteristic of said phosphorus substrate, thereby permitting the calculation of both substrate concentrations.

2. A method for the simultaneous determination of the concentration of calcium and phosphorus substrates in a single test sample with a single reagent system, said method comprising the steps of:
adding to said test sample in a single step said single reagent system containing a chromophore for each of said calcium and phosphorus substrates, each chromophore being selected such that it is capable of contributing to a discrete electromagnetic radiation signal for the respective calcium or phosphorus substrate, wherein the determination of calcium does not interfere with the determination of phosphorus, and the determination of phosphorus does not interfere with the determination of calcium;
reacting said test sample with said reagent system whereby said calcium and phosphorus substrates react simultaneously with their respective chromophores;
monitoring changes in absorbance or fluorescence of the resulting reaction mixture at two or more wavelengths at least one of which which is characteristic of said calcium substrate and at least one of which is characteristic of said phosphorus substrate, thereby permitting the calculation of both substrate concentrations.

3. The method of Claim 2 wherein said reagent system comprises an azo dye in a concentration sufficient to allow an endpoint determination of calcium; and an acidic aqueous solution comprising a molybdate salt and detergent in a concentration sufficient to dissolve serum components and allow an endpoint determination of phosphorus.

4. The method of Claim 3 wherein said reagent system comprises chlorophosphonazo-III in a concentration sufficient to allow an endpoint determination of calcium; and sodium or ammonium molybdate, sulfuric acid in an amount sufficient to adjust said reagent system to about pH 2, and detergent in a concentration sufficient to dissolve serum components and allow an endpoint determination of phosphorus.

5. The method of Claim 3 wherein said reagent system comprises chlorophosphonazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

6. The method of Claim 3 wherein said reagent system comprises arsenazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

7. A reagent system for the simultaneous determination of calcium and phosphorus comprising an azo dye in a concentration sufficient to allow an endpoint determination of calcium; and an acidic aqueous solution comprising a molybdate salt and detergent in a concentration sufficient to dissolve serum components and allow an endpoint determination of phosphorus.

8. A reagent system according to Claim 7 comprising chlorophosphonazo-III in a concentration sufficient to allow an endpoint determination of calcium; and sodium or ammonium molybdate, sulfuric acid in an amount sufficient to adjust said reagent system to about pH 2, and detergent in a concentration sufficient to dissolve serum components and allow an endpoint determination of phosphorus.

9. A reagent system according to Claim 7 comprising chlorophosphonazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

10. A reagent system according to Claim 7 comprising arsenazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

## Claims (Claims for the following Contracting State(s): ES)

1. A method for the simultaneous determination of the concentration of calcium and phosphorus substrates in a single test sample with a single reagent system, said method comprising the steps of:
adding to said test sample in a single step said single reagent system containing a reactant for each of said calcium and phosphorus substrates, each reactant being selected such that it is capable of contributing to a discrete electromagnetic radiation signal for the respective calcium or phosphorus substrate, wherein the determination of calcium does not interfere with the determination of phosphorus, and the determination of phosphorus does not interfere with the determination of calcium;
reacting said test sample with said reagent system whereby said calcium and phosphorus substrates react simultaneously with their respective reactants;
monitoring changes in absorbance or fluorescence of the resulting reaction mixture at two or more wavelengths at least one of which which is characteristic of said calcium substrate and at least one of which is characteristic of said phosphorus substrate, thereby permitting the calculation of both substrate concentrations.

2. A method for the simultaneous determination of the concentration of calcium and phosphorus substrates in a single test sample with a single reagent system, said method comprising the steps of:
adding to said test sample in a single step said single reagent system containing a chromophore for each of said calcium and phosphorus substrates, each chromophore being selected such that it is capable of contributing to a discrete electromagnetic radiation signal for the respective calcium or phosphorus substrate, wherein the determination of calcium does not interfere with the determination of phosphorus, and the determination of phosphorus does not interfere with the determination of calcium;
reacting said test sample with said reagent system whereby said calcium and phosphorus substrates react simultaneously with their respective chromophores;
monitoring changes in absorbance or fluorescence of the resulting reaction mixture at two or more wavelengths at least one of which which is characteristic of said calcium substrate and at least one of which is characteristic of said phosphorus substrate, thereby permitting the calculation of both substrate concentrations.

3. The method of Claim 2 wherein said reagent system comprises an azo dye in a concentration sufficient to allow an endpoint determination of calcium; and an acidic aqueous solution comprising a molybdate salt and detergent in a concentration sufficient to dissolve serum components and allow an endpoint determination of phosphorus.

4. The method of Claim 3 wherein said reagent system comprises chlorophosphonazo-III in a concentration sufficient to allow an endpoint determination of calcium; and sodium or ammonium molybdate, sulfuric acid in an amount sufficient to adjust said reagent system to about pH 2, and detergent in a concentration sufficient to dissolve serum components and allow an endpoint determination of phosphorus.

5. The method of Claim 3 wherein said reagent system comprises chlorophosphonazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

6. The method of Claim 3 wherein said reagent system comprises arsenazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

7. A reagent system for the simultaneous determination of calcium and phosphorus comprising an azo dye in a concentration sufficient to allow an endpoint determination of calcium; and an acidic aqueous solution comprising a molybdate salt and detergent in a concentration sufficient to dissolve serum components and allow an endpoint determination of phosphorus.

8. A reagent system according to Claim 7 comprising chlorophosphonazo-III in a concentration sufficient to allow an endpoint determination of calcium; and sodium or ammonium molybdate, sulfuric acid in an amount sufficient to adjust said reagent system to about pH 2, and detergent in a concentration sufficient to dissolve serum components and allow an endpoint determination of phosphorus.

9. A reagent system according to Claim 7 comprising chlorophosphonazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

10. A reagent system according to Claim 7 comprising arsenazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

11. A process for making a reagent system comprising combining, in an aqueous solution, an azo dye in a concentration sufficient to allow an endpoint determination of calcium; and a molybdate salt, an acid and detergent in a concentration sufficient to dissolve serum components and allow an endpoint determination of phosphorus.

12. A process according to Claim 11 for making a reagent system comprising combining, in an aqueous solution, chlorophosphonazo-III in a concentration sufficient to allow an endpoint determination of calcium; and sodium or ammonium molybdate, sulfuric acid in an amount sufficient to adjust said reagent system to about pH 2, and detergent in a concentration sufficient to dissolve serum components and allow an endpoint determination of phosphorus.

13. A process according to Claim 11 for making a reagent system comprising combining, in an aqueous solution, chlorophosphonazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

14. A process according to Claim 11 for making a reagent system comprising combining, in an aqueous solution, arsenazo-III in a concentration sufficient to allow an endpoint determination of calcium; and ammonium molybdate, acetic acid/NaOH in an amount sufficient to adjust said reagent system to about pH 4, and detergent in a concentration sufficient to dissolve serum components and allow an endpoint determination of phosphorus.

## Patentansprüche (Patentansprüche für folgende(n) Vertragsstaat(en): AT, BE, CH, DE, FR, GB, IT, LI, NL)

1. Ein Verfahren für die simultane Bestimmung der Konzentration von Calcium- und Phosphorsubstraten in einer einzelnen Testprobe mit einem einzigen Reagentiensatz, wobei das Verfahren aus folgenden Schritten besteht:
Zufügen des einzigen Reagentiensatzes, bestehend aus einem Reaktanden für jedes Calcium- und Phosphorsubstrat, zu der Testprobe in einem einzigen Schritt, wobei jeder Reaktant so gewählt ist, daß er in der Lage ist, ein diskretes elektromagnetisches Strahlungssignal für das entsprechende Calcium- oder Phosphorsubstrat zu liefern, wobei die Calciumbestimmung die Phosphorbestimmung nicht beeinflußt, und die Phosphorbestimmung die Calciumbestimmung nicht beeinflußt;
Umsetzung der Testprobe mit dem Reagentiensatz, wobei die Calcium- und Phosphorsubstrate gleichzeitig mit ihrem entsprechenden Reaktanden reagieren;
Überwachung der Änderungen der Extinktion oder der Fluoreszenz des resultierenden Reaktionsgemisches bei zwei oder mehreren Wellenlängen, von denen mindestens eine charakteristisch für das Calciumsubstrat und mindestens eine charakteristisch für das Phosphorsubstrat ist, wodurch die Berechnung der beiden Substratkonzentrationen ermöglicht wird.

2. Ein Verfahren für die simultane Bestimmung der Konzentration von Calcium- und Phosphorsubstraten in einer einzelnen Testprobe mit einem einzigen Reagentiensatz, wobei das Verfahren aus folgenden Schritten besteht:
Zufügen des einzigen Reagentiensatzes, der ein Chromophor für jedes Calcium- und Phosphorsubstrat umfaßt, zu der Testprobe in einem einzigen Schritt, wobei jedes Chromophor so gewählt ist, daß es in der Lage ist, einen Beitrag zu einem elektromagnetischen Strahlungssignal des entsprechenden Calcium- oder Phosphorsubstrates zu liefern, wobei die Calciumbestimmung die Phosphorbestimmung nicht beeinflußt, und die Phosphorbestimmung die Calciumbestimmung nicht beeinflußt;
Umsetzung der Testprobe mit dem Reagentiensatz, wobei die Calcium- und Phosphorsubstrate gleichzeitig mit ihrem entsprechenden Chromophor reagieren;
Überwachung der Änderungen der Extinktion oder der Fluoreszenz des resultierenden Reaktionsgemischs bei zwei oder mehreren Wellenlängen, von denen mindestens eine charakteristisch für das Calciumsubstrat, und mindestens eine charakteristisch für das Phosphorsubstrat ist, wodurch die Berechnung der beiden Substratkonzentrationen ermöglicht wird.

3. Das Verfahren nach Anspruch 2, wobei der Reagentiensatz einen Azofarbstoff in einer ausreichenden Konzentration enthält, um die Endpunktbestimmung von Calcium zu ermöglichen, und eine saure wäßrige Lösung mit einem Molybdatsalz und einem Detergens in einer ausreichenden Konzentration, um die Serumkomponenten zu lösen und eine Endpunktbestimmung von Phosphor zu ermöglichen.

4. Das Verfahren nach Anspruch 3, wobei der Reagentiensatz Chlorphosphonazo-III in einer ausreichenden Konzentration enthält, um die Endpunktbestimmung von Calcium zu ermöglichen, und Natrium- oder Ammoniummolybdat und Schwefelsäure in einer ausreichenden Menge, um den Reagentiensatz auf etwa pH 2 einzustellen, und ein Detergens in einer ausreichenden Konzentration, um die Serumkomponenten zu lösen und eine Endpunktbestimmung von Phosphor zu ermöglichen.

5. Das Verfahren nach Anspruch 3, wobei der Reagentiensatz Chlorphosphonazo-III in einer ausreichenden Konzentration enthält, um die Endpunktbestimmung von Calcium zu ermöglichen, und Ammoniummolybdat und Essigsäure/NaOH in einer ausreichenden Menge, um den Reagentiensatz auf etwa pH 4 einzustellen, und ein Detergens in einer ausreichenden Konzentration, um die Serumkomponenten zu lösen und eine Endpunktbestimmung von Phosphor zu ermöglichen.

6. Das Verfahren nach Anspruch 3, wobei der Reagentiensatz Arsenazo-III in einer ausreichenden Konzentration enthält, um die Endpunktbestimmung von Calcium zu ermöglichen, und Ammoniummolybdat und Essigsäure/NaOH in einer ausreichenden Menge, um den Reagentiensatz auf etwa pH 4 einzustellen, und ein Detergens in einer ausreichenden Konzentration, um die Serumkomponenten zu lösen und eine Endpunktbestimmung von Phosphor zu ermöglichen.

7. Ein Reagentiensatz für die simultane Bestimmung von Calcium und Phosphor, umfassend einen Azofarbstoff in einer ausreichenden Konzentration, um die Endpunktbestimmung von Calcium zu ermöglichen und eine saure wäßrige Lösung, die Molybdatsalz und Detergens in einer ausreichenden Konzentration enthält, um die Serumkomponenten zu lösen und eine Endpunktbestimmung von Phosphor zu ermöglichen.

8. Ein Reagentiensatz nach Anspruch 7 mit Chlorphosphonazo-III in einer ausreichenden Konzentration, um die Endpunktbestimmung von Calcium zu ermöglichen, der Natrium- oder Ammoniummolybdat und Schwefelsäure in einer ausreichenden Menge enthält, um den Reagentiensatz auf etwa pH 2 einzustellen, und der ein Detergens in einer ausreichenden Konzentration enthält, um die Serumkomponenten zu lösen und eine Endpunktbestimmung von Phosphor zu ermöglichen.

9. Ein Reagentiensatz nach Anspruch 7 mit Chlorphosphonazo-III in einer ausreichenden Konzentration, um die Endpunktbestimmung von Calcium zu ermöglichen, der Ammoniummolybdat und Essigsäure/NaOH in einer ausreichenden Menge enthält, um den Reagentiensatz auf etwa pH 4 einzustellen, und der ein Detergens in einer ausreichenden Konzentration enthält, um die Serumkomponenten zu lösen und eine Endpunktbestimmung von Phosphor zu ermöglichen.

10. Ein Reagentiensatz nach Anspruch 7 mit Arsenazo-III in einer ausreichenden Konzentration, um die Endpunktbestimmung von Calcium zu ermöglichen, der Ammoniummolybdat und Essigsäure/NaOH in einer ausreichenden Menge enthält, um den Reagentiensatz auf etwa pH 4 einzustellen, und der ein Detergens in einer ausreichenden Konzentration enthält, um die Serumkomponenten zu lösen und eine Endpunktbestimmung von Phosphor zu ermöglichen.

## Patentansprüche (Patentansprüche für folgende(n) Vertragsstaat(en): ES)

1. Ein Verfahren für die simultane Bestimmung der Konzentration von Calcium- und Phosphorsubstraten in einer einzelnen Testprobe mit einem einzigen Reagentiensatz, wobei die Methode aus folgenden Schritten besteht:
Zufügen des einzigen Reagentiensatzs, bestehend aus einem Reaktanden für jedes Calcium- und Phosphorsubstrat, zu der Testprobe in einem einzigen Schritt, wobei jeder Reaktant so gewählt ist, daß er in der Lage ist, ein diskretes elektromagnetisches Strahlungssignal für das entsprechende Calcium- oder Phosphorsubstrat zu liefern, worin die Calciumbestimmung die Phosphorbestimmung nicht beeinflußt, und die Phosphorbestimmung die Calciumbestimmung nicht beeinflußt;
Umsetzung der Testprobe mit dem Reagentiensatz, wobei die Calcium- und Phosphorsubstrate gleichzeitig mit ihrem entsprechenden Reaktanden reagieren;
Überwachung der Änderungen der Extinktion oder der Fluoreszenz des resultierenden Reaktionsgemisches bei zwei oder mehreren Wellenlängen, von denen mindestens eine charakteristisch für das Calciumsubstrat und mindestens eine charakteristisch für das Phosphorsubstrat ist, wodurch die Berechnung der beiden Substratkonzentrationen ermöglicht wird.

2. Ein Verfahren für die simultane Bestimmung der Konzentration von Calcium- und Phosphorsubstraten in einer einzelnen Testprobe mit einem einzigen Reagentiensatz, wobei die Methode aus folgenden Schritten besteht:
Zufügen des einzigen Reagentiensatzes, beinhaltend ein Chromophor für jedes Calcium- und Phosphorsubstrat, zu der Testprobe in einem einzigen Schritt, wobei jedes Chromophor so gewählt ist, daß es in der Lage ist, einen Beitrag zu einem elektromagnetischen Strahlungssignal des entsprechenden Calcium- oder Phosphorsubstrates zu liefern, wobei die Calciumbestimmung die Phosphorbestimmung nicht beeinflußt, und die Phosphorbestimmung die Calciumbestimmung nicht beeinflußt;
Umsetzung der Testprobe mit dem Reagentiensatz, wobei die Calcium- und Phophorsubstrate gleichzeitig mit ihrem entsprechenden Chromophor reagieren;
Überwachung der Änderungen der Extinktion oder der Fluoreszenz des resultierenden Reaktionsgemisches bei zwei oder mehreren Wellenlängen, von denen mindestens eine charakteristisch für das Calciumsubstrat und mindestens eine charakteristisch für das Phosphorsubstrat ist, wodurch die Berechnung der beiden Substratkonzentrationen ermöglicht wird.

3. Das Verfahren nach Anspruch 2, wobei der Reagentiensatz einen Azofarbstoff in einer ausreichenden Konzentration enthält, um die Endpunktbestimmung von Calcium zu ermöglichen, und eine saure wäßrige Lösung mit einem Molybdatsalz und einem Detergens in einer ausreichenden Konzentration, um die Serumkomponenten zu lösen und eine Endpunktbestimmung von Phosphor zu ermöglichen.

4. Das Verfahren nach Anspruch 3, wobei der Reagentiensatz Chlorphosphonazo-III in einer ausreichenden Konzentration enthält, um die Endpunktbestimmung von Calcium zu ermöglichen, und Natrium- oder Ammoniummolybdat und Schwefelsäure in einer ausreichenden Menge, um den Reagentiensatz auf etwa pH 2 einzustellen, und ein Detergens in einer ausreichenden Konzentration, um die Serumkomponenten zu lösen und eine Endpunktbestimmung von Phosphor zu ermöglichen.

5. Das Verfahren nach Anspruch 3, wobei der Reagentiensatz Chlorphosphonazo-III in einer ausreichenden Konzentration enthält, um die Endpunktbestimmung von Calcium zu ermöglichen und Ammoniummolybdat und Essigsäure/NaOH in einer ausreichenden Menge, um den Reagentiensatz auf etwa pH 4 einzustellen, und ein Detergens in einer ausreichenden Konzentration, um die Serumkomponenten zu lösen und eine Endpunktbestimmung von Phosphor zu ermöglichen.

6. Das Verfahren nach Anspruch 3, wobei der Reagentiensatz Arsenazo-III in einer ausreichenden Konzentration enthält, um die Endpunktbestimmung von Calcium zu ermöglichen, und Ammoniummolybdat und Essigsäure/NaOH in einer ausreichenden Menge, um das Reagentiensatz auf etwa pH 4 einzustellen, und ein Detergens in einer ausreichenden Konzentration, um die Serumkomponenten zu lösen und eine Endpunktbestimmung von Phosphor zu ermöglichen.

7. Ein Reagentiensatz für die simultane Bestimmung von Calcium und Phosphor, der einen Azofarbstoff in einer ausreichenden Konzentration umfaßt, um die Endpunktbestimmung von Calcium zu ermöglichen und eine saure wäßrige Lösung, die ein Molybdatsalz und Detergens in einer ausreichenden Konzentration enthält, um die Serumkomponenten zu lösen und eine Endpunktbestimmung von Phosphor zu ermöglichen.

8. Ein Reagentiensatz nach Anspruch 7 mit Chlorphosphonazo-III in einer ausreichenden Konzentration, um die Endpunktbestimmung von Calcium zu ermöglichen, der Natrium- oder Ammoniummolybdat und Schwefelsäure in einer ausreichenden Menge enthält, um den Reagentiensatz auf etwa pH 2 einzustellen, und der ein Detergens in einer ausreichenden Konzentration enthält, um die Serumkomponenten zu lösen und eine Endpunktbestimmung von Phosphor zu ermöglichen.

9. Ein Reagentiensatz nach Anspruch 7 mit Chlorphosphonazo-III in einer ausreichenden Konzentration, um die Endpunktbestimmung von Calcium zu ermöglichen, der Ammoniummolybdat und Essigsäure/NaOH in einer ausreichenden Menge enthält, um den Reagentiensatz auf etwa pH 4 einzustellen, und der ein Detergens in einer ausreichenden Konzentration enthält, um die Serumkomponenten zu lösen und eine Endpunktbestimmung von Phosphor zu ermöglichen.

10. Ein Reagentiensatz nach Anspruch 7 mit Arsenazo-III in einer ausreichenden Konzentration, um die Endpunktbestimmung von Calcium zu ermöglichen, der Ammoniummolybdat und Essigsäure/NaOH in einer ausreichenden Menge enthält, um den Reagentiensatz auf etwa pH 4 einzustellen, und der ein Detergens in einer ausreichenden Konzentration enthält, um die Serumkomponenten zu lösen und eine Endpunktbestimmung von Phosphor zu ermöglichen.

11. Ein Verfahren zur Bereitung eines Reagentiensatzes, welches das Zusammengeben in wässriger Lösung eines Azofarbstoffes, in einer zur Endpunktbestimmung von Calcium ausreichenden Konzentration, eines Molybdatsalzes, einer Säure und eines Detergentiums in einer zur Auflösung der Serumkomponenten ausreichenden und die Endpunktbestimmung des Phosphors erlaubenden Konzentration, umfaßt.

12. Ein Verfahren nach Anspruch 11 zur Bereitung eines Reagentiensatzes, welches das Zusammengeben in wässriger Lösung von Chlorphosphonazo-III, in einer für die Endpunktbestimmung von Calcium ausreichenden Konzentration, und von Natrium- oder Ammoniummolybdat und von Schwefelsäure in einer geeigneten Menge, um den Reagentiensatz auf einen pH von ungefähr 2 einzustellen, und von Detergens in einer ausreichenden Menge, um die Serumkomponenten aufzulösen und die Endpunktbestimmung von Phosphor zu erlauben, umfaßt.

13. Ein Verfahren nach Anspruch 11 zur Bereitung eines Reagentiensatzes, welches das Zusammengeben in wässriger Lösung von Chlorphosphonazo-III, in einer ausreichenden Konzentration, um die Endbestimmung des Calciums zu erlauben, und von Ammoniummolybdat, Essigsäure/NaOH, in einer zur Einstellung des Reagentiensatzes auf ungefähr pH 4 ausreichenden Menge, und von Detergens in einer zur Auflösung der Serumkomponenten ausreichenden und die Endpunktbestimmung des Phosphors erlaubenden Konzentration, umfaßt.

14. Ein Verfahren nach Anspruch 11 zur Bereitung eines Reagentiensatzes, welches das Zusammengeben in wässriger Lösung von Arsenazo-III in einer ausreichenden Konzentration, um die Endbestimmung des Calciums zu erlauben, und von Ammoniummolybdat, Essigsäure/NaOH, in einer zur Einstellung des Reagentiensatzes auf ungefähr pH 4 ausreichenden Menge, und von Detergens, in einer zur Auflösung der Serumkomponenten ausreichenden und die Endpunktbestimmung des Phosphors erlaubenden Konzentration, umfaßt.

## Revendications (Revendications pour l'(les) Etat(s) contractant(s) suivant(s): AT, BE, CH, DE, FR, GB, IT, LI, NL)

1. Méthode pour la détermination simultanée de la concentration des substrats calcium et phosphore dans un même échantillon à tester avec un système de réactifs unique, ladite méthode comprenant les étapes :
de l'addition dudit échantillon à tester dans une seule étape audit système de réactifs unique contenant un réactif pour chacun desdits substrats calcium et phosphore, chaque réactif étant sélectionné de façon à être capable de contribuer à un signal de rayonnement électromagnétique discret pour les substrats respectifs calcium et phosphore, la détermination du calcium n'interférant pas avec la détermination du phosphore, et la détermination du phosphore n'interférant pas avec la détermination du calcium;
de la réaction dudit échantillon à tester avec ledit système de réactifs dans laquelle lesdits substrats calcium et phosphore réagissent simultanément avec leurs réactifs respectifs;
du suivi des modifications de l'absorbance ou de la fluorescence du mélange réactionnel obtenu à deux ou plusieurs longueurs d'onde, au moins une d'entre elles étant caractéristique dudit substrat calcium et au moins une d'entre elles étant caractéristique dudit substrat phosphore, permettant ainsi le calcul des concentrations des deux substrats.

2. Méthode pour la détermination simultanée de la concentration des substrats calcium et phosphore dans un même échantillon à tester avec un système de réactifs unique, ladite méthode comprenant les étapes :
de l'addition dudit échantillon à tester dans une seule étape audit système de réactifs unique contenant un chromophore pour chacun desdits substrats calcium et phosphore, chaque chromophore étant sélectionné de façon à être capable de contribuer à un signal de rayonnement électromagnétique discret pour les substrats respectifs calcium et phosphore, la détermination du calcium n'interférant pas avec la détermination du phosphore, et la détermination du phosphore n'interférant pas avec la détermination du calcium;
de la réaction dudit échantillon à tester avec ledit système de réactifs dans laquelle lesdits substrats calcium et phosphore réagissent simultanément avec leurs chromophores respectifs;
du suivi des modifications de l'absorbance ou de la fluorescence du mélange réactionnel obtenu à deux ou plusieurs longueurs d'onde, au moins une d'entre elles étant caractéristique dudit substrat calcium et au moins une d'entre elles étant caractéristique dudit substrat phosphore, permettant ainsi le calcul des concentrations des deux substrats.

3. Méthode de la revendication 2, dans laquelle ledit système de réactifs comprend un colorant azoïque en une concentration suffisante pour permettre un dosage du calcium au point final de la réaction ; et une solution aqueuse acide comprenant un sel de molybdate et un détergent en une concentration suffisante pour dissoudre les constituants du sérum et permettre une détermination du phosphore au point final de la réaction.

4. Méthode de la revendication 3, dans laquelle ledit système de réactifs comprend du chlorophosphonazo-III en une concentration suffisante pour permettre un dosage du calcium au point final de la réaction ; et du molybdate de sodium ou d'ammonium, de l'acide sulfurique en une quantité suffisante pour ajuster le pH dudit système de réactifs à environ 2, et un détergent en une concentration suffisante pour dissoudre les constituants du sérum et permettre une détermination du phosphore au point final de la réaction.

5. Méthode de la revendication 3, dans laquelle ledit système de réactifs comprend du chlorophosphonazo-III en une concentration suffisante pour permettre un dosage du calcium au point final de la réaction ; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

6. Méthode de la revendication 3, dans laquelle ledit système de réactifs comprend de l'arsénazo-III en une concentration suffisante pour permettre un dosage du calcium au point final de la réaction ; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

7. Système de réactifs pour le dosage simultané du calcium et du phosphore comprenant un colorant azoïque en une concentration suffisante pour permettre un dosage du calcium au point final de la réaction ; une solution aqueuse acide comprenant un sel de molybdate et un détergent en une concentration suffisante pour dissoudre les constituants du sérum et permettre une détermination du phosphore au point final de la réaction.

8. Système de réactifs selon la revendication 7 comprenant du chlorophosphonazo-III en une concentration suffisante pour permettre un dosage du calcium au point final de la réaction ; et du molybdate de sodium ou d'ammonium, de l'acide sulfurique en une quantité suffisante pour ajuster le pH dudit système de réactifs à environ 2, et un détergent en une concentration suffisante pour dissoudre les constituants du sérum et permettre une détermination du phosphore au point final de la réaction.

9. Système de réactifs selon la revendication 7, comprenant du chlorophosphonazo-III en une concentration suffisante pour permettre un dosage du calcium au point final de la réaction ; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

10. Système de réactifs selon la revendication 7, comprenant de l'arsénazo-III en une concentration suffisante pour permettre un dosage du calcium au point final de la réaction; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

## Revendications (Revendications pour l'(les) Etat(s) contractant(s) suivant(s): ES)

1. Méthode pour la détermination simultanée de la concentration des substrats calcium et phosphore dans un même échantillon à tester avec un système de réactifs unique, ladite méthode comprenant les étapes :
de l'addition dudit échantillon à tester dans une seule étape audit système de réactifs unique contenant un réactif pour chacun desdits substrats calcium et phosphore, chaque réactif étant sélectionné de façon à être capable de contribuer à un signal de rayonnement électromagnétique discret pour les substrats respectifs calcium et phosphore, la détermination du calcium n'interférant pas avec la détermination du phosphore, et la détermination du phosphore n'interférant pas avec la détermination du calcium;
de la réaction dudit échantillon à tester avec ledit système de réactifs dans laquelle lesdits substrats calcium et phosphore réagissent simultanément avec leurs réactifs respectifs;
du suivi des modifications de l'absorbance ou de la fluorescence du mélange réactionnel obtenu à deux ou plusieurs longueurs d'onde, au moins une d'entre elles étant caractéristique dudit substrat calcium et au moins une d'entre elles étant caractéristique dudit substrat phosphore, permettant ainsi le calcul des concentrations des deux substrats.

2. Méthode pour la détermination simultanée de la concentration des substrats calcium et phosphore dans un même échantillon à tester avec un système de réactifs unique, ladite méthode comprenant les étapes :
de l'addition dudit échantillon à tester dans une seule étape audit système de réactifs unique contenant un chromophore pour chacun desdits substrats calcium et phosphore, chaque chromophore étant sélectionné de façon à être capable de contribuer à un signal de rayonnement électromagnétique discret pour les substrats respectifs calcium et phosphore, la détermination du calcium n'interférant pas avec la détermination du phosphore, et la détermination du phosphore n'interférant pas avec la détermination du calcium;
de la réaction dudit échantillon à tester avec ledit système de réactifs dans laquelle lesdits substrats calcium et phosphore réagissent simultanément avec leurs chromophores respectifs;
du suivi des modifications de l'absorbance ou de la fluorescence du mélange réactionnel obtenu à deux ou plusieurs longueurs d'onde, au moins une d'entre elles étant caractéristique dudit substrat calcium et au moins une d'entre elles étant caractéristique dudit substrat phosphore, permettant ainsi le calcul des concentrations des deux substrats.

3. Méthode de la revendication 2, dans laquelle ledit système de réactifs comprend un colorant azoïque en une concentration suffisante pour permettre un dosage du calcium au point final de la réaction ; et une solution aqueuse acide comprenant un sel de molybdate et un détergent en une concentration suffisante pour dissoudre les constituants du sérum et permettre une détermination du phosphore au point final de la réaction.

4. Méthode de la revendication 3, dans laquelle ledit système de réactifs comprend du chlorophosphonazo-III en une concentration suffisante pour permettre un dosage du calcium au point final de la réaction ; et du molybdate de sodium ou d'ammonium, de l'acide sulfurique en une quantité suffisante pour ajuster le pH dudit système de réactifs à environ 2, et un détergent en une concentration suffisante pour dissoudre les constituants du sérum et permettre une détermination du phosphore au point final de la réaction.

5. Méthode de la revendication 3, dans laquelle ledit système de réactifs comprend du chlorophosphonazo-III en une concentration suffisante pour permettre un dosage du calcium au point final de la réaction ; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

6. Méthode de la revendication 3, dans laquelle ledit système de réactifs comprend de l'arsénazo-III en une concentration suffisante pour permettre un dosage du calcium au point final de la réaction; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

7. Système de réactifs pour le dosage simultané du calcium et du phosphore comprenant un colorant azoïque en une concentration suffisante pour permettre un dosage du calcium au point final de la réaction ; une solution aqueuse acide comprenant un sel de molybdate et un détergent en une concentration suffisante pour dissoudre les constituants du sérum et permettre une détermination du phosphore au point final de la réaction.

8. Système de réactifs selon la revendication 7 comprenant du chlorophosphonazo-III en une concentration suffisante pour permettre un dosage du calcium au point final de la réaction ; et du molybdate de sodium ou d'ammonium, de l'acide sulfurique en une quantité suffisante pour ajuster le pH dudit système de réactifs à environ 2, et un détergent en une concentration suffisante pour dissoudre les constituants du sérum et permettre une détermination du phosphore au point final de la réaction.

9. Système de réactifs selon la revendication 7, comprenant du chlorophosphonazo-III en une concentration suffisante pour permettre un dosage du calcium au point final de la réaction; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

10. Système de réactifs selon la revendication 7, comprenant de l'arsénazo-III en une concentration suffisante pour permettre un dosage du calcium au point final de la réaction; et du molybdate d'ammonium, de l'acide acétique/NaOH en une quantité suffisante pour ajuster le pH dudit système de réactifs à environ 4, et un détergent en une concentration suffisante pour dissoudre les constituants du sérum et permettre une détermination du phosphore au point final de la réaction.

11. Procédé pour réaliser un système de réactifs comprenant la combinaison, dans une solution aqueuse, d'un colorant azoïque en une concentration suffisante pour permettre un dosage du calcium au point final de la réaction ; et d'un sel de molybdate, d'un acide et d'un détergent en une concentration suffisante pour dissoudre les constituants du sérum et permettre une détermination du phosphore au point final de la réaction.

12. Procédé selon la revendication 11 pour réaliser un système de réactifs comprenant la combinaison, dans une solution aqueuse, de chlorophosphonazo-III en une concentration suffisante pour permettre un dosage du calcium au point final de la réaction ; et de molybdate de sodium ou d'ammonium, d'acide sulfurique en une quantité suffisante pour ajuster le pH dudit système de réactifs à environ 2, et de détergent en une concentration suffisante pour dissoudre les constituants du sérum et permettre une détermination du phosphore au point final de la réaction.

13. Procédé selon la revendication 11, pour réaliser un système de réactifs, comprenant la combinaison, dans une solution aqueuse, de chlorophosphonazo-III en une concentration suffisante pour permettre un dosage du calcium au point final de la réaction ; et de molybdate d'ammonium, d'acide acétique/NaOH en une quantité suffisante pour ajuster lepH dudit système de réactifs à environ 4, et d'un détergent en une concentration suffisante pour dissoudre les constituants du sérum et permettre une détermination du phosphore au point final de la réaction.

14. Procédé selon la revendication 11, pour réaliser un système de réactifs, comprenant la combinaison, dans une solution aqueuse, d'arsénazo-III en une concentration suffisante pour permettre un dosage du calcium au point final de la réaction ; et de molybdate d'ammonium, d'acide acétique/NaOH en une quantité suffisante pour ajuster le pH dudit système de réactifs à environ 4, et d'un détergent en une concentration suffisante pour dissoudre les constituants du sérum et permettre une détermination du phosphore au point final de la réaction.
